# EUROPEAN PATENT SPECIFICATION

(11) **EP 2 997 933 B1**
(45) Date of publication and mention of the grant of the patent: **11.10.2017**
(21) Application number: 15275134.3
(22) Date of filing: 11.05.2015
(51) Int. Cl.: A61F 2/01, A61B 17/12

(54) **SPRING LOCK IMPLANTABLE VASCULAR DEVICE**
IMPLANTIERBARE VASKULÄRE SCHNAPPVERSCHLUSSVORRICHTUNG
DISPOSITIF VASCULAIRE IMPLANTABLE DE VERROU À RESSORT

(30) Priority: 19.09.2014 GB 201416594
(43) Date of publication of application: 23.03.2016
(73) Proprietor: Cook Medical Technologies LLC, Bloomington, IN 47404 (US)
(72) Inventor: Dela, Christian, 2500 Valby (DK)
(74) Representative: Williams Powell

(56) References cited:
- WO-A1-2005/046783
- WO-A1-2014/002087
- JP-A- H08 257 031
- US-A1- 2002 116 024

## Description

### Technical Field

The present invention relates to an implantable medical device, for example a vascular filter such as a vena cava filter. The teachings herein may also apply to an occluder or other implantable medical device.

### Background Art

Various designs of vascular filter are known in the art, many of which can be implanted endoluminally into a patient from a remote percutaneous entry point. Such filters may be left in the patient long term or permanently, for example for the treatment of deep vein thrombosis or for filtering clots in the vena cava. It is also known to use such filters on a short term basis, for instance during a medical procedure or in the course of a temporary ailment.

It is important to maintain such vascular filters correctly in position in the vessel during their use, and specifically to stop filter migration caused, for example, by blood pressure and/or vessel movement. It is also important to minimise the risk of tilting of the filter, which can cause loss of filtering function and leakage of blood around the filter. In order to minimise the risk of positional migration or tilting, filters often have anchoring elements such as barbs which are arranged in use to pierce into the wall of the vessel, thereby to hold the filter in position. Filters may also include orientation elements, such as a set of secondary filter legs which hold the filter aligned with the vessel. Barbs can cause vessel damage, particularly if they are able to be pulled out of the vessel wall as a result of changes in blood pressure or vessel movement. It is also possible for such barbs to penetrate deeply into the vessel wall, in some instances to pierce all the way through the thickness of the vessel wall. While some filter anchoring systems are intended to pierce through the entire thickness of the vessel wall, in many cases this is not desirable.

Some examples of vascular filters are disclosed in US-8,409,239, US-2013/0218195, US-7,056,286 and US-2006/0015137. WO 2005/046783 A1 discloses a medical device anchor and delivery system. Japanese publication number JP H08257031 discloses a filter.

### Disclosure of the Invention

The present invention seeks to provide an improved implantable medical device and in particular an improved vascular filter or occluder. The device may be a vena cava filter.

According to an aspect of the present invention, there is provided an implantable vascular device including a hub and a plurality of legs, each leg including a proximal end coupled to the hub and extending distally and radially outwardly of the hub to a distal end of the leg, the legs creating a generally conical form to at least a portion of the device; wherein the distal end of each leg includes an anchoring element, each anchoring element including a loop of resilient material curving so as to cross the leg and having a piercing member at an end of the loop and extending beyond the loop in a direction generally radially outwardly of the device, each loop being a sprung element and providing a vessel abutment surface for limiting the penetration of the piercing member into a vessel wall.

Each anchoring element may be an extension of its respective leg. Preferably, each anchoring element is formed as a bent or curved distal portion of its respective leg.

In the preferred embodiment, each loop is disposed in a generally radial orientation; such that in use each loop is substantially perpendicular to the vessel wall at the point of contact.

Advantageously, each loop is resiliently compressible to a smaller loop diameter. The advantage of a loop having such characteristics is that the resiliency of the loop can accommodate changes in the diameter or shape of the vessel and in particular provide for variation to the fixing of the piercing members, enabling better and adaptable fixation of the piercing members to the vessel wall.

Each piercing element may be substantially straight. They could, in other embodiments, be curved or bent.

Preferably, each loop has a diameter of at least 3 millimetres. Each piercing member may have a length of between 1 millimetres and 2.54 millimetres.

Preferably, the device has a longitudinal dimension and each piercing member extends in a direction substantially perpendicular to the longitudinal dimension. In other words, the piercing members may be disposed perpendicularly to the longitudinal axis of the vessel wall, as well as radially outwardly.

Each anchoring element is preferably sufficiently resilient to be able to be straightened for deployment and filter retrieval.

The anchoring elements are advantageously unconstrained relative to their associated legs, which means that they will immediately adopt their "at rest", or non-biased, configurations as soon as released from a delivery catheter or sheath.

The device may be provided with a plurality of elongate support elements, each support element including a proximal end coupled to the hub and extending distally from and radially outwardly of the hub to a distal end, the support elements creating a generally second conical form to the filter; wherein the support elements have a length less than a length of the legs and having a point of maximum radius spaced from a point of maximum radius of the legs.

The support elements may be formed from spring steel, although in other embodiments they may be made of other materials such as a shape memory material, typically Nitinol.

In an embodiment, there is provided a flexible coupling element disposed between the legs and the hub. The flexible coupling element may be a braided or coiled tubular element.

At least the legs are preferably formed from a shape memory material, an example being a shape memory alloy such as Nitinol.

The device is preferably a vascular filter or occluder. It may, for instance, be a vena cava filter.

Other features and advantages will become apparent form the description which follows and in the accompanying drawings.

### Brief Description of the Drawings

Embodiments of the present invention are described below, by way of example only, with reference to the accompanying drawings, in which:
Figure 1 is a side elevational view in schematic form of an embodiment of vascular filter according to the teachings herein;
Figure 2 is an enlarged view of the distal end of a filter leg of the filter of Figure 1;
Figure 3 is an enlarged view of a part of the filter of Figure 1;
Figure 4 is a part cross-sectional view showing a side elevation of the filter assembly of Figure 1 disposed in a delivery catheter or sheath;
Figure 5 is a part cross-sectional view showing the filter of Figures 1 to 4 deployed in a vessel;
Figure 6 is an end view of the filter of Figure 5 in the vessel;
Figure 7 is a view of a part of another embodiment of vascular filter; and
Figure 8 is a side elevational view of another embodiment of vascular filter.

### Description of the Preferred Embodiments

The accompanying drawings are schematic only. It is to be understood that the dimensions and proportions of the various components of the devices shown in the drawings are not to scale or in proportion relative to one another. It is also to be understood that the drawings depict only the principal components of the device shown therein and that other conventional elements and components of the device which are not central to understanding the teachings herein have been omitted for the sake of clarity.

The embodiments described below with reference to the accompanying drawings are directed to a vascular filter, typically a vena cava filter. It is to be understood, though, that the teachings herein can be used for other implantable medical devices including, for example but not limited to, occlusion devices. An occlusion device could be formed from a structure similar to that shown in the accompanying drawings and described below, for instance by covering the filter legs with an occlusion barrier of an type which will be known to a person skilled in the art.

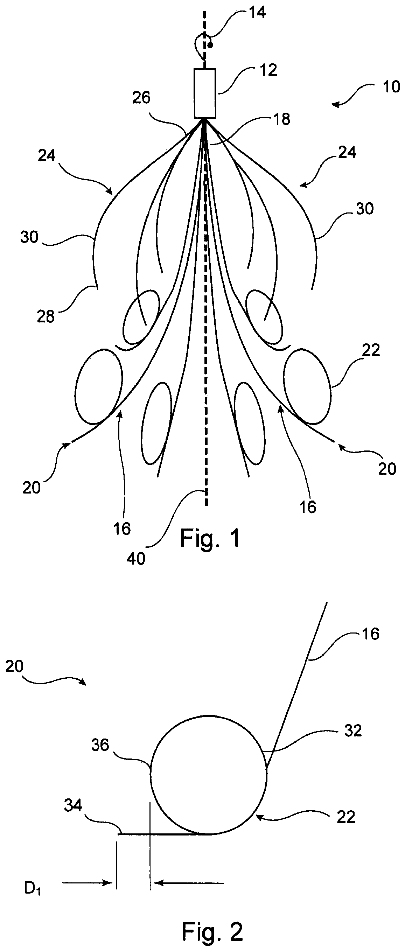

Referring first to Figure 1, this shows in schematic form an embodiment of filter assembly 10 which has what could be described as a generally conical form. The filter assembly 10 includes a hub 12, which is typically a tube of metal or metal alloy, usefully of the same material as the filter legs, although could equally be made of a different metal, metal alloy or of a polymer material. At one end of the hub 12 there is provided an attachment element 14, which in this embodiment is in the form of a hook, used both in the deployment of the filter assembly 10 and also in the retrieval of filter assembly 10, in a manner which will be familiar to the skilled person. The attachment element 14 is typically made of metal.

The filter assembly 10 includes a set of filter legs 16, each of which has a first end 80 attached to the hub 12 and a second end 20 including an anchoring element 22 described in further detail below. The second ends 20 of the filter legs 16 extend distally from and radially outwardly of the hub 12, such that the filter legs 16 in combination have a generally conical form widening from the hub 12 to the second ends 20. The extremities 20 in practice abut against the vessel wall, as is described in further detail below.

The filter assembly 10 includes a series of support elements 24 which are also, in this embodiment, in the form of legs. Each support element or leg 24 includes a first end 26 which is attached to the hub 12 and a second end 28 spaced distally of the hub 12 and which is radially expanded relative to the hub. Each support element or leg 24 curves outwardly from the hub 12 towards a vessel contact point 30, before curving inwardly again towards the second ends 28.

The filter legs 16 and/or support elements 24 may be made of wire members attached to the hub 12, for example by fitting into a lumen of a tubular hub 12 and then being welded, bonded or otherwise fixed into the hub 12. In another embodiment, the legs 16 and/or support elements 24 may be laser cut from a tubing forming the hub 12, such that the hub and filter legs and/or support elements are formed from a common length of tubing.

In the preferred embodiment, the filter legs 16 are made of a shape memory material, such as a shape memory alloy. Preferably, the legs 16 are made of Nitinol. The support elements 24 are in a preferred embodiment made of steel, such as spring steel, although they may equally be made of shape memory material such as Nitinol.

With reference now to Figure 2, this shows the second end 20 of one of the filter legs 16. It will be appreciated that each of the filter legs 16 has the structure shown in Figure 2 in the preferred embodiment.

The anchoring element 22 includes a loop 32 and a piercing member 34. In the preferred embodiment, the anchoring element 22 is formed by coiling the end 20 of the filter leg 16 to form a single-turn loop 32 with the extremity of the end 22 being left uncoiled and straight so as to form the piercing member 34. The wire of the loop 32 is free to slide over itself, that is it is not bonded or fixed to itself.

Each piercing member 34 extends by a distance D₁ beyond the loop 32 and in particular beyond a vessel abutment surface 36 of the loop 32. This distance D₁ in practice represents the penetration distance of the piercing element 34 into the vessel wall.

It is preferred that each loop has a diameter of at least 3 mm and that each piercing member has a length of between 1 mm and 2.54 mm.

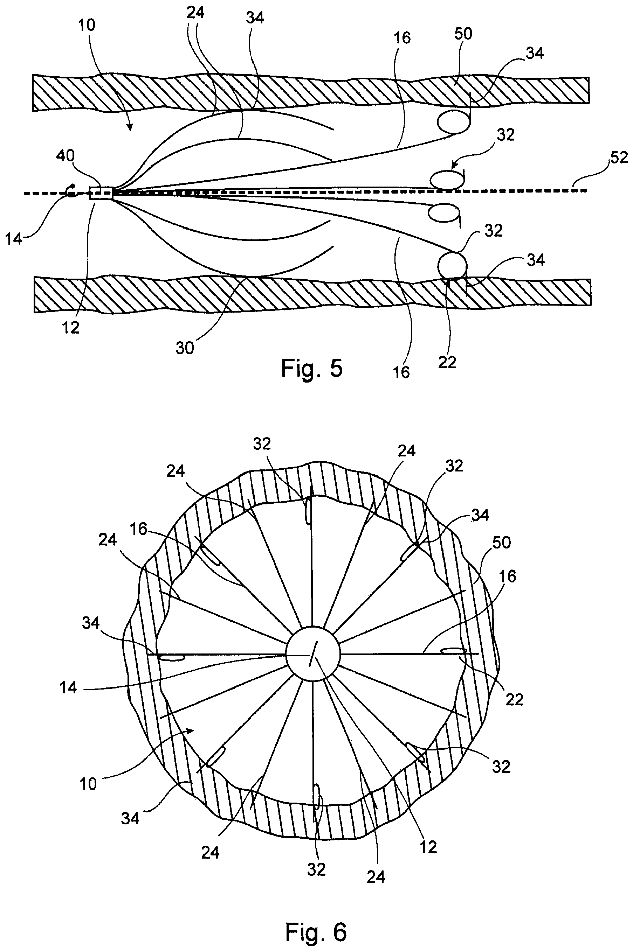

As will become apparent below, in particular with regard to Figures 5 and 6, the loops 32 are disposed radially from a centre line 40 of the filter assembly 10, as shown in Figure 1. As a result, described below, the loops 32 will abut the vessel wall substantially perpendicularly to the vessel wall (along radii to the conical form of the device). The piercing elements 34 preferably also extend radially of the centre line 40 and also substantially perpendicularly to that centre line 40 and therefore perpendicular to the longitudinal direction of the filter assembly 10.

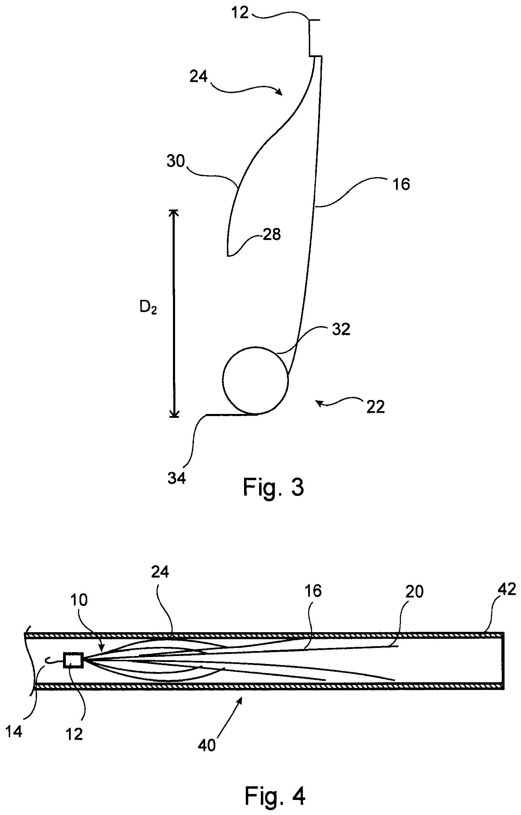

Referring now to Figure 3, this shows in schematic form a part of the filter assembly 10 and in particular of one of the filter legs 16 and of one of the support elements 24. The filter legs 16 and support elements 24 of the assembly 10 provide two sets of vessel contact points, one being at the piercing elements 34 and the other at the points 30 of greatest radius of the support elements 24. These two contact points 30 and 34 are spaced from one another by a distance D₂ in the longitudinal direction 40 of the filter assembly 10. The provision of these two vessel contact points 30 and 34 assists in giving the filter assembly 10 orientational stability within a vessel. In other words, they ensure that the filter assembly maintains the correct orientation within the vessel and such that the piercing elements 34 are arranged in a plane perpendicular to the longitudinal direction of the vessel, thereby ensuring optimum coupling of the filter assembly 10 to the vessel and optimum patency to the vessel wall to minimise any flow leakage around the outside of the filter assembly 10.

The example of filter assembly 10 of Figure 1 has six filter legs 16 and six support elements 24. A person skilled in the art will appreciate that the number of filter legs and support elements can vary from that shown and will generally be dependent upon the size of the vessel and the degree of filtration which is desired to be achieved by the assembly 10. Where greater filtration is desired, there may be provided a greater number of filter legs 16 and in some embodiments also of the support elements 24, which will reduce the size of the gaps between adjacent filter legs and support elements, thereby to capture smaller particles in the blood stream.

When the filter assembly 10 or at least the legs 16 are formed of a shape memory material, the shape of the filter legs 16 shown in Figures 2 and 3 represents the memorised shape of the device, the same applying to the support elements 24 when these are also made of shape memory material. Therefore, when the filter assembly 10 has transitioned through the Af temperature of the shape memory material, this will tend towards the shape shown in Figures 2 and 3.

Referring now to Figure 4, this shows the filter assembly 10 disposed in radially compressed form within a delivery catheter or sheath 40 of an introducer assembly. The introducer assembly will include a deployment mechanism located within the catheter 40, which holds the medical device 10 in position within the catheter 40 and which deploys the medical device into the vessel of a patient. It will also typically include an external manipulation unit for operating the various elements of the introducer assembly. As all of these components and their functions are commonplace in the art, they are not shown in Figure 4 and not described further herein. The skilled person will already be able to identify the suitable components and devices from those commonly available.

It is to be appreciated that in practice the sheath or catheter 42 will have a smaller diameter than the depiction of Figure 4, in order to hold the filter assembly 10 in a tighter contracted state than that shown in Figure 4, thereby to give the introducer assembly a smaller footprint. The schematic depiction of Figure 4 better shows the contracted condition of the filter assembly 10, where it can be seen that the filter legs 16 are straightened, such that the anchoring elements 22 are also substantially straight. In the embodiments in which the legs 16 are made of a shape memory material, the filter assembly 10 is generally maintained below the transition temperature of the material during assembly and transportation, which enables the filter legs 16 and in particular the loop anchoring elements 22 to be readily straightened and held in that straightened configuration. On deployment, the filter assembly 10 will be heated to above the shape memory transition (Af) temperature (typically just below body temperature), which will cause the ends 20 of the legs 16 to regain their memorised shape, that is to regain the looped ends 32 and piercing elements 34.

The medical device 10 is typically deployed from the catheter 40 either by pushing or pulling it out from the distal end of the catheter, in dependence upon the chosen deployment mechanism. On deployment of the medical device 10, that is upon its release from within the catheter 40 and the transition of the legs 16 beyond the Af transition temperature, the legs will coil into the loops 32 shown in Figures 1, 2 and 3. This is typically done only once the filter assembly 10 has been located in the desired position within the patient's vasculature.

Referring now to Figure 5, this shows the filter assembly 10 fully deployed within a patient's vessel 50. The vessel may be the vena cava or any other vessel within the human or animal body.

As can be seen in Figure 5, the filter assembly 10 has its longitudinal axis 40 aligned with the longitudinal axis 52 of the vessel 50 and such that the piercing members 34 all lie in a plane substantially perpendicular to the longitudinal axis 52 of the vessel 50. This is achieved by the provision of the two vessel contact points 30 (of the support elements 24) and 36 (of the loops 32 of the anchoring elements 22). The piercing members 34 pierce into the thickness of the vessel wall 50, by the distance D₁ shown in Figure 2. The outermost surface, or vessel abutment surface 36, of the loops 32 prevents the piercing elements 34 embedding too deeply into the vessel wall 50, as well as pressing against the vessel wall. With reference also to Figure 6, this shows the filter assembly 10 of Figure 5 when viewed into the vessel 50 from the side of the hook 14, that is from the left of Figure 5. Figure 6 shows the support elements 24 interspersed between the filter legs 16 and coming into abutment with the internal surface of the vessel 50, at the first contact points 30 shown in Figure 5. The filter legs 32, in particular their second ends 20, also come into contact with the internal surface of the vessel wall 50 and in a manner in which each loop 32 is substantially perpendicular to the tangent to the vessel wall 50 at the point at which the loops contact the vessel wall, in other words along radii of the vessel. The piercing elements 34 extend radially into the vessel wall, as will be particularly apparent from Figure 6.

The loops 32 of the anchoring elements 22 have a plurality of advantages over the art. First, they act as stop elements to prevent the piercing members 34 piercing too far into the thickness of the vessel wall 50. Secondly, they are flexible allowing the loops 32 to flex during movement of the vessel wall and also against the opening force at the distal ends 20 of the filter legs 16 caused by the springiness of the legs. This assists in holding the distal ends 20 of the filter legs 16 in position in the vessel and also in maintaining the piercing members 34 embedded within the vessel wall. This flexibility can in particular retain the filter assembly 10 in place as the vessel wall moves outwardly and inwardly with changes in blood pressure and also as it moves as a result of natural movement of the patient.

The resiliency of the loops 32 can also assist in maintaining the piercing elements 34 perpendicular to the vessel wall during movement of the vessel, with the result that the filter is better able to stay in place.

Furthermore, it is not necessary to provide any rearwardly extending hooks on the piercing elements 34, as is required with some other designs of medical device.

In practice, once the filter assembly has been implanted within a patient, there can be expected to be tissue growth over the distal ends 20 of the filter legs 16 and in particular over at least a part of the anchoring elements 22. However, as these are flexible, the filter assembly can still be removed from the vessel. The hook 14 can be attached to a retrieval mechanism which is then used to pull the filter assembly in the direction of the hook 14. As the assembly 10 is pulled, the loops 32 will straighten and slide out of any ingrown vessel tissue, thereby making the retrieval of the filter assembly readily achievable. This contrasts with some prior art assemblies where the piercing elements are provided with rearwardly extending hooks to hold the piercing elements in the vessel wall, the latter only being removable by tearing from the vessel wall.

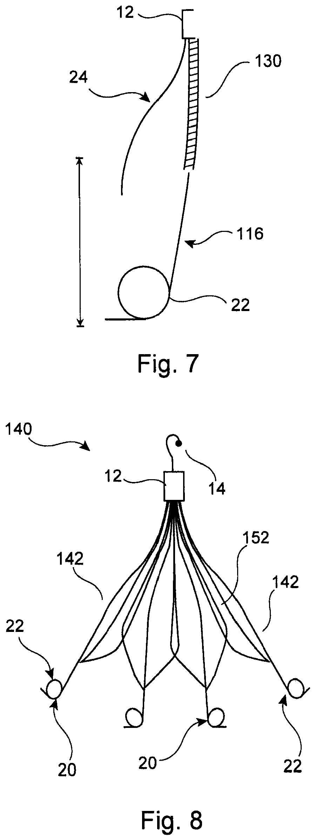

Referring now to Figure 7, this shows a modified form of filter leg 116 which can be used in place of the filter leg 16 of the filter assembly 10 shown in the previous drawings. The filter leg 116 is virtually the same as the filter leg 16 previously disclosed, save for the provision of a section 130 of more flexible material, in one example being a braided or coiled flexible tubing coupled between the hub 12 and the wire forming the distal section of the filter leg 16. The section 130 adds flexibility to the filter legs, useful in improving the tilting resistance of the filter assembly and in maintaining it centred within the vessel.

Figure 8 shows another embodiment of filter assembly 140 having a plurality of filter legs 142, each terminating in anchoring elements 22 as with the previously described embodiments, but in which there are provided secondary filter struts or legs 152 which extend from an intermediate position just short of the distal ends 20 of the filter legs up to the hub 12. The struts 152 have the purpose of creating filtering barriers in the spaces between adjacent filter legs 142, thereby to increase the filtering efficacy of the assembly 140.

It is to be appreciated that it is not necessary for the piercing elements 34 to be disposed precisely perpendicularly to the vessel wall as they could be disposed at an angle to the perpendicular. For example, the piercing elements 34 could in some embodiments point towards the hub end of the filter assembly. In another embodiment they could point in the other direction, that is away from the hub 12, although this is preferably by no more than a modest angle of a few degrees.

Similarly, it is not necessary for the loops 32, or for the piercing elements 34, to be disposed precisely radially relative to the centre line 40 of the filter assembly and similarly precisely in the vessel 50, as they (loops 32 and/or piercing elements 34) could be at a slight angle to this, for instance by a few degrees or so.

Although in the preferred embodiment the loops 32 are a single turn of the wire forming the legs 22, in other embodiments the loops 32 could be made from multiple turns of wire.

As explained above, although the described embodiments are all directed to a filter assembly, the teachings herein are not limited to vascular filters. The teachings could equally apply to occluder elements, in which case there may be provided an impervious or substantially impervious occluding barrier coupled to the filter legs and/or the support elements.

The device could be configured for deployment over a guide wire, in which case when in the delivery catheter or sheath 40 (shown in Figure 4) a guide wire may pass alongside the hub 12 and through the mass of filter legs and support elements. In another embodiment a guide wire could pass through a lumen within the hub 12 and through the centreline 40 of the filter assembly 10.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

## Claims

1. An implantable vascular device (10) including a hub (12) and a plurality of legs (16), each leg including a proximal end (18) coupled to the hub (12) and extending distally and radially outwardly of the hub (12) to a distal end (20) of the leg, the legs creating a generally conical form to at least a portion of the device; wherein the distal end (20) of each leg includes an anchoring element (22), each anchoring element including a loop (32) of resilient material curving so as to cross the leg (16) and having a piercing member (34) at an end of the loop and extending beyond the loop in a direction generally radially outwardly of the device, each loop (32) being a sprung element and providing a vessel abutment surface (36) for limiting the penetration of the piercing member (34) into a vessel wall.

2. A device (10) according to claim 1, wherein each anchoring element (22) is an extension of its respective leg (16).

3. A device (10) according to claim 1 or 2, wherein each anchoring element (22) is formed as a bent distal part of its respective leg (16).

4. A device (10) according to any preceding claim, wherein each loop (32) is disposed in a generally radial orientation.

5. A device (10) according to any preceding claim, wherein each loop (32) is resiliently compressible to a smaller loop diameter.

6. A device (10) according to any preceding claim, wherein each piercing element (34) is substantially straight.

7. A device (10) according to any preceding claim, wherein each loop (32) has a diameter of at least 3 millimetres.

8. A device (10) according to any preceding claim, wherein each piercing member (34) has a length of between 1 millimetres and 2.54 millimetres.

9. A device (10) according to any preceding claim, wherein the device has a longitudinal dimension and each piercing member (34) extends in a direction substantially perpendicular to the longitudinal dimension.

10. A device (10) according to any preceding claim, wherein each anchoring element (22) is sufficiently resilient to be able to be straightened for deployment and filter retrieval.

11. A device (10) according to any preceding claim, wherein each anchoring element (22) is unconstrained relative to its associated leg (16).

12. A device (10) according to any preceding claim, including a plurality of elongate support elements (24), each support element including a proximal end (26) coupled to the hub (12) and extending distally from and radially outwardly of the hub to a distal end (28), the support elements (24) creating a generally second conical form to the filter; wherein the support elements (24) have a length less than a length of the legs (16) and having a point of maximum radius (30) spaced from a point of maximum radius of the legs.

13. A device (10) according to claim 12, wherein the support elements (24) are support legs.

14. A device (10) according to claim 12 or 13, wherein the support elements (24) are formed from steel.

15. A device (10) according to any preceding claim, including a flexible coupling element (130) disposed between the legs (16) and the hub (12).

16. A device (10) according to claim 15, wherein the flexible coupling element (130) is a braided or coiled tubular element.

17. A device (10) according to any preceding claim, wherein at least the legs (16) are formed from a shape memory material.

## Patentansprüche

1. Implantierbare vaskuläre Vorrichtung (10), die einen Ansatz (12) und eine Vielzahl von Schenkeln (16) einschließt, wobei jeder Schenkel ein proximales Ende (18) einschließt, das an den Ansatz (12) gekoppelt ist und sich distal und radial auswärts von dem Ansatz (12) zu einem distalen Ende (20) des Schenkels erstreckt, wobei die Schenkel eine allgemein konische Form für mindestens einen Abschnitt der Vorrichtung erzeugen; wobei das distale Ende (20) jedes Schenkels ein Verankerungselement (22) einschließt, jedes Verankerungselement eine Schleife (32) aus elastischem Material einschließt, die sich so krümmt, dass sie den Schenkel (16) kreuzt, und mit einem Durchstechelement (34) am Ende der Schleife versehen ist, das sich über die Schleife hinaus in eine Richtung allgemein radial auswärts der Vorrichtung erstreckt, wobei jede Schleife (32) ein gefedertes Element ist und eine Gefäßanliegeoberfläche (36) bereitstellt, um das Eindringen des Durchstechelements (34) in eine Gefäßwand zu begrenzen.

2. Vorrichtung (10) nach Anspruch 1, wobei jedes Verankerungselement (22) eine Verlängerung seines jeweiligen Schenkels (16) ist.

3. Vorrichtung (10) nach Anspruch 1 oder 2, wobei jedes Verankerungselement (22) als ein gebogenes distales Teil seines jeweiligen Schenkels (16) gebildet ist.

4. Vorrichtung (10) nach einem der vorhergehenden Ansprüche, wobei jede Schleife (32) in einer allgemein radialen Orientierung angeordnet ist.

5. Vorrichtung (10) nach einem der vorhergehenden Ansprüche, wobei jede Schleife (32) elastisch zu einem kleineren Schleifendurchmesser komprimierbar ist.

6. Vorrichtung (10) nach einem der vorhergehenden Ansprüche, wobei jedes Durchstechelement (34) im Wesentlichen gerade ist.

7. Vorrichtung (10) nach einem der vorhergehenden Ansprüche, wobei jede Schleife (32) einen Durchmesser von mindestens 3 Millimetern aufweist.

8. Vorrichtung (10) nach einem der vorhergehenden Ansprüche, wobei jedes Durchstechelement (34) eine Länge zwischen 1 Millimeter und 2,54 Millimetern aufweist.

9. Vorrichtung (10) nach einem der vorhergehenden Ansprüche, wobei die Vorrichtung eine Längsabmessung aufweist und jedes Durchstechelement (34) sich in eine Richtung erstreckt, die im Wesentlichen senkrecht zu der Längsabmessung ist.

10. Vorrichtung (10) nach einem der vorhergehenden Ansprüche, wobei jedes Verankerungselement (22) ausreichend elastisch ist, um zur Ausbringung und Rückholung des Filters begradigt zu werden.

11. Vorrichtung (10) nach einem der vorhergehenden Ansprüche, wobei jedes Verankerungselement (22) relativ zu seinem dazugehörigen Schenkel (16) ungekoppelt ist.

12. Vorrichtung (10) nach einem der vorhergehenden Ansprüche, einschließend eine Vielzahl länglicher Trägerelemente (24), wobei jedes Trägerelement ein proximales Ende (26) einschließt, das an den Ansatz (12) gekoppelt ist und sich distal zu und radial auswärts von dem Ansatz zu einem distalen Ende (28) erstreckt, wobei die Trägerelemente (24) eine allgemein zweite konische Form für den Filter erzeugen; wobei die Trägerelemente (24) eine Länge aufweisen, die kleiner als eine Länge der Schenkel (16) ist, und einen Punkt des maximalen Radius (30) aufweisen, der von einem Punkt des maximalen Radius der Schenkel beabstandet ist.

13. Vorrichtung (10) nach Anspruch 12, wobei die Trägerelemente (24) Trägerschenkel sind.

14. Vorrichtung (10) nach Anspruch 12 oder 13, wobei die Trägerelemente (24) aus Stahl gebildet sind.

15. Vorrichtung (10) nach einem der vorhergehenden Ansprüche, die ein flexibles Kupplungselement (130) einschließt, das zwischen den Schenkeln (16) und dem Ansatz (12) angeordnet ist.

16. Vorrichtung (10) nach Anspruch 15, wobei das flexible Kupplungselement (130) ein geflochtenes oder gewickeltes Rohrelement ist.

17. Vorrichtung (10) nach einem der vorhergehenden Ansprüche, wobei mindestens die Schenkel (16) aus einem Formgedächtnismaterial gebildet sind.

## Revendications

1. Dispositif vasculaire implantable (10) comprenant un moyeu (12) et une pluralité de jambes (16), chaque jambe comprenant une extrémité proximale (18) accouplée au moyeu (12) et s'étendant distalement et radialement vers l'extérieur du moyeu (12) jusqu'à une extrémité distale (20) de la jambe, les jambes donnant une forme dans l'ensemble conique à au moins une partie du dispositif ; dans lequel l'extrémité distale (20) de chaque jambe comprend un élément d'ancrage (22), chaque élément d'ancrage comprenant une boucle (32) de matière élastique se courbant de façon à croiser la jambe (16) et comportant un élément de perçage (34) à une extrémité de la boucle et s'étendant au-delà de la boucle dans une direction dans l'ensemble radiale extérieure du dispositif, chaque boucle (32) étant un élément à ressort et assurant une surface de butée (36) contre un vaisseau pour limiter la pénétration de l'élément de perçage (34) dans la paroi du vaisseau.

2. Dispositif (10) selon la revendication 1, dans lequel chaque élément d'ancrage (22) est une extension de sa jambe (16) respective.

3. Dispositif (10) selon la revendication 1 ou 2, dans lequel chaque élément d'ancrage (22) est constitué comme une partie distale courbée de sa jambe (16) respective.

4. Dispositif (10) selon l'une quelconque des revendications précédentes, dans lequel chaque boucle (32) est disposée selon une orientation dans l'ensemble radiale.

5. Dispositif (10) selon l'une quelconque des revendications précédentes, dans lequel chaque boucle (32) peut être comprimée élastiquement jusqu'à un diamètre de boucle plus petit.

6. Dispositif (10) selon l'une quelconque des revendications précédentes, dans lequel chaque élément de perçage (34) est sensiblement droit.

7. Dispositif (10) selon l'une quelconque des revendications précédentes, dans lequel chaque boucle (32) a un diamètre d'au moins 3 millimètres.

8. Dispositif (10) selon l'une quelconque des revendications précédentes, dans lequel chaque élément de perçage (34) a une longueur comprise entre 1 millimètre et 2,54 millimètres.

9. Dispositif (10) selon l'une quelconque des revendications précédentes, dans lequel le dispositif a une dimension longitudinale et chaque élément de perçage (34) s'étend dans une direction sensiblement perpendiculaire à ladite dimension longitudinale.

10. Dispositif (10) selon l'une quelconque des revendications précédentes, dans lequel chaque élément d'ancrage (22) est suffisamment élastique pour pouvoir être redressé pour le déploiement la récupération du filtre.

11. Dispositif (10) selon l'une quelconque des revendications précédentes, dans lequel chaque élément d'ancrage (22) est non contraint par rapport à sa jambe (16) associée.

12. Dispositif (10) selon l'une quelconque des revendications précédentes, comprenant une pluralité d'éléments de support (24) allongés, chaque élément de support comprenant une extrémité proximale (26) accouplée au moyeu (12) et s'étendant distalement du moyeu et radialement vers l'extérieur de celui-ci jusqu'à une extrémité distale (28), les éléments de support (24) donnant une seconde forme dans l'ensemble conique au filtre ; dans lequel les éléments de support (24) ont une longueur inférieure à la longueur des jambes (16) et ont un point (30) de rayon maximal espacé du point de rayon maximal des jambes.

13. Dispositif (10) selon la revendication 12, dans lequel les éléments de support (24) sont des jambes de support.

14. Dispositif (10) selon la revendication 12 ou 13, dans lequel les éléments de support (24) sont constitués d'acier.

15. Dispositif (10) selon l'une quelconque des revendications précédentes, comprenant un élément d'accouplement (130) flexible est disposé entre les jambes (16) et le moyeu (12).

16. Dispositif (10) selon la revendication 15, dans lequel l'élément d'accouplement (130) flexible est un élément tubulaire tressé ou spiralé.

17. Dispositif (10) selon l'une quelconque des revendications précédentes, dans lequel au moins les jambes (16) sont constituées d'une matière à mémoire de forme.
